Europäisches Patentamt

European Patent Office

Office européen des brevets

(11) Publication number: **0 073 850**
**B1**

# EUROPEAN PATENT SPECIFICATION

(45) Date of publication of patent specification: **28.08.85**

(51) Int. Cl.⁴: **A 61 K 6/00**

(21) Application number: **81106903.8**

(22) Date of filing: **03.09.81**

(54) Dental prosthesis adhesive.

(43) Date of publication of application:
**16.03.83 Bulletin 83/11**

(45) Publication of the grant of the patent:
**28.08.85 Bulletin 85/35**

(84) Designated Contracting States:
**BE CH DE FR GB IT LI NL SE**

(56) References cited:
**DE-A-1 050 508**
**US-A-3 440 065**

(73) Proprietor: **Richardson GmbH**
**H.-S.-Richardson-Strasse Postfach 1661**
**D-6080 Gross-Gerau (DE)**

(72) Inventor: **Wienecke, Horst, Dr.**
**H.-S. Richardson-Strasse**
**D-6080 Gross-Gerau (DE)**

(74) Representative: **Goddar, Heinz J., Dr.**
**FORRESTER & BOEHMERT Widenmayerstrasse 4/I**
**D-8000 München 22 (DE)**

Courier Press, Leamington Spa, England.

EP 0 073 850 B1

## Description

The invention relates to a dental prosthesis adhesive formed from particles of one or more adhesive substances as well as to a process of producing same.

It is generally known to produce dental prosthesis adhesives using chemical or natural substances imparting an adhesive action and which are used individually, mixed with one another or distributed in carrier substances. These dental prosthesis adhesives give a natural prosthesis fit, which generally lasts for several hours.

Cellulose containing denture adhesive is known from US—A—3440065, where a mixture of about 30 to about 60% of topically acceptable adhesive hydrocolloid, about 1.5 to 20% by weight of powdered insoluble absorptive cellulose material and about 35 to about 65% petrolatum is disclosed.

This known denture adhesive did have a good adhesive action between denture and gingiva and palate.

From DE—B—1050508 there is known a denture adhesive in powder form, comprising plant-gums, alginates or pectinates, comprising between 1 to 15% per weight of the product greasy or waxy material, having a solid appearance at room temperature.

In general, a maximum duration of the adhesive action is desired which should last over a maximum day period of up to 17 or more hours. The adhesives known from the above referenced state of the art show a long adhesive action of the day, but it would be very difficult for the prosthesis wearer to remove the denture during the period in question whenever he would wish to do so because the adhesive action is extremely powerful. It is also extremely difficult to clean such adhesives from the dental prosthesis.

Thus, the problem of the invention is to provide a dental prosthesis adhesive permitting a reliable fit over long periods, whilst still making it possible to remove the prosthesis without difficulty.

According to the invention, this problem is solved in that at least a part of the particles of the adhesive substance or substances are provided with a coating which slowly dissolves in the saliva.

The invention thus relates to a dental prosthesis adhesive formed from particles of one or more adhesive substances, characterized in that at least a portion of the particles of the adhesive substance or substances are provided with a coating which dissolves slowly in saliva.

The invention also relates to a process for producing a dental prosthesis adhesive, characterized by coating particles of adhesive substance or substances with a coating which slowly dissolves in the saliva and/or in ingestive liquids and mixing the coated particles of the adhesive substance or substances with particles of uncoated adhesive substance or substances.

Accordingly, the invention teaches to treat adhesive substances which ensure a reliable hold of up to 8 hours and more and which do not impair the removal of the denture in a manner providing a delaying action. Unlike the disadvantageous prior art prosthesis adhesives in which all the adhesive substances are simultaneously used in the active state, in the dental prosthesis adhesive according to the invention the release of the adhesion-imparting substances takes place in time-controlled manner, in that in part the adhesion-imparting substances are provided with a coating which slowly dissolves in the saliva and/or in ingestive liquids and when dissolved releases unconsumed adhesive and comes into action after the "consumption" or release or washing away of the already consumed adhesive.

The adhesive substances can be chemically defined compounds, for example sodium carboxymethyl cellulose, Karaya gum, guar gum, tragacanth, polyethylene oxide polymers; copolymers of maleic anhydride with lower alkyl-vinylether and mixed partial salts thereof polyacrylamide, mixtures thereof, natural substances or mixtures thereof or also mixtures with chemical substances. Optionally the denture adhesive composition may contain flavours, colorants, preservatives, fillers and other common denture adhesive additives. The dental prosthesis adhesive according to the invention is produced by coating part of the adhesive substances with a varyingly thick protective film, and saliva or ingested liquids successively release the adhesive by dissolving the coating. The coating on the adhesive particles can be formed by the most varied substances such as e.g. ethyl cellulose, saccharose monostearate, gum arabic, cellulose acetate phthalate, acrylates, methycrylate and shellac.

The coating agent, which is e.g. used as a film, can be formed by substances which are insoluble or sparingly soluble in water, their sparing solubility only occurring within specific pH-ranges.

Conventionally, between approximately 10 and 90% of the particles of adhesive substances are coated, coating taking place e.g. by spraying adhesive substance particles with the coating agent dissolved in a solvent.

Conventionally, between approximately 10 and 90% of the particles of adhesive substances used are coated and initially the uncoated portion brings about the adhesion of the prosthesis. After washing out this uncoated portion, the remaining adhesive is slowly released after a time which can be predetermined by the choice of coating the thickness thereof, so that a much longer overall adhesive period can be achieved than would be possible with uncoated adhesive. As a result of the process according to the invention, the conventional adhesion time can be extended by several additional hours. However, no difficulties are encountered if it is possibly necessary in the meantime to remove the prosthesis, because the adhesion of the adhesive is always the same as with normal adhesives being effective for shorter periods.

In addition, no difficulties are encountered in cleaning the prosthesis because the conventional, easily removable prosthesis adhesives can be used.

Further features and advantages of the invention can be gathered from the claims and the following description of performance examples.

### Example 1
### Preparation of Adhesive Powder

150 kg of sodium carboxymethyl cellulose (particle size max. 0.6 mm) are sprayed in a fluidized bed procedure (fluidized bed drier like AEROMATIC or GLATT) with 120 kg of a 6.5% methacrylate isopropanol solution, resulting in a coating with a thickness of 2—5 micrometer. Spraying and drying are conducted in one step according to the conventional fluidized bed procedure. The yield is about 160 kg sodium carboxymethyl cellulose coated by a film.

40 parts by weight of the coated sodium carboxymethyl cellulose are well mixed with 60 parts by weight of non coated sodium carboxymethyl cellulose. The resulting pulverulent mixture can be used as an adhesive powder for dental prosthesis with a considerably longer adhesion period than conventional similar adhesives.

### Example 2
### Preparation of a Semifluid Dental Prosthesis Adhesive

An adhesive powder prepared in the manner described in example 1 is incorporated into a semifluid carrier constituted by a mixture of paraffin and glycol or polyethyleneglycol and glycerol. The resulting highly viscous liquid imparts good adhesion to a dental prosthesis for a longer period than the conventional similar adhesives.

### Example 3
### Preparation of a Prosthesis Adhesive Powder

150 kg sodium carboxymethyl cellulose (particle size max. 0.6 mm) are sprayed in a fluidized bed procedure (fluidized bed drier like AEROMATIC or GLATT) with 60 kg of a 6.5% ethylalcoholic ethylcellulose solution, resulting in a coating with a thickness of 2—5 micrometer. Spraying and drying are conducted in one step according to the conventional fluidized bed procedure. The yield is about 160 kg NaCMC, the particles thereof being coated with an ethylcellulose film.

50 parts by weight of the coated NaCMC (sodium carboxymethyl cellulose) are well mixed with 50 parts by weight of uncoated sodium carboxymethyl cellulose. The resultant powder can be used as an adhesive powder for dental prosthesis having a longer effective period than conventional similar adhesives.

### Example 4
### Preparation of a Dental Prosthesis Cream

The adhesive powder mixture described in example 3 is incorporated into a creamy carrier formed from petrolatum and liquid petrolatum and paraffin oil. The adhesive cream provides a satisfactory adhesive action for a longer period, when applied to a dental prosthesis, than conventional similar adhesives.

Further, suitable flavorants, such as peppermint oil, or the like can be added to the denture adhesive compositions to improve the taste.

**Claims**

1. Dental prosthesis adhesive formed from particles of one or more adhesive substances, characterized in that at least a portion of the particles of the adhesive substance or substances are provided with a coating which dissolves slowly in saliva.

2. Dental prosthesis adhesive according to claim 1, characterized in that the coating for the particles of the adhesive substance contains one or more substances selected from ethyl cellulose, saccharose monostearate, gum arabic, cellulose acetate phthalate, acrylate, methacylate or shellac.

3. Dental prosthesis adhesive according to either of the claims 1 and 2, characterized in that the particles of the adhesive substance at least partly contains sodium carboxymethyl cellulose.

4. Dental prosthesis adhesive according to one of the preceding claims, characterized in that the coating is sparingly soluble in water.

5. Dental prosthesis adhesive according to one of the claims 1 to 3, characterized in that the coating is insoluble in water.

6. Dental prosthesis according to one of the claims 1 to 3, characterized in that the coating is soluble at a pH-value from 3,5 to 9,0.

7. Dental prosthesis adhesive according to one of the preceding claims, characterized in that 10 to 90% of the adhesive substance used is coated.

8. Dental prosthesis according to claim 1, characterized in that sodium carboxymethyl cellulose coated with ethyl cellulose is used as the coated adhesive substance.

9. Dental prosthesis adhesive according to claim 8, characterized in that 50% coated sodium carboxymethyl cellulose is used as the adhesive substance.

10. Dental prosthesis adhesive according to one of the preceding claims, characterized in that the at least partly coated particles of adhesive substance or substances are embedded in a creamy carrier substance.

11. Dental prosthesis adhesive according to claim 10, characterized in that the creamy carrier substance contains petrolatum and liquid petrolatum and paraffin oil.

12. Dental prosthesis adhesive according to one of the claims 1 to 9, characterized in that the at least partly coated particles of adhesive substance or substances are embedded in a semifluid carrier substance.

13. Dental prosthesis adhesive according to claim 12, characterized in that the semifluid carrier substance is a mixture of paraffin and glycerol or polyethylene glycol and glycerol.

14. Process for the preparation of a dental prosthesis adhesive, characterized by coating particles of adhesive substance or substances with a coating which slowly dissolves in the saliva and/or ingestive liquids and mixing the coated particles of the adhesive substance or substances with uncoated particles of adhesive substance or substances.

15. Process according to claim 14, characterized in that the mixture is prepared from 90% to 10% of coated particles of an adhesive substance and 90 to 10% of noncoated particles of the adhesive substance.

16. Process according to claim 15, characterized in that the mixture is embedded in a carrier.

17. Process according to claim 16, characterized in that the carrier is semifluid.

18. Process according to claim 17, characterized in that the semifluid carrier is a mixture of paraffin and glycerol and polyethylen glycol and glycerol.

19. Process according to claim 17, characterized in that the carrier is creamy.

20. Process according to claim 19, characterized in that the creamy carrier is a mixture of petrolatum and liquid petrolatum and paraffin oil.

21. Process according to one of the claims 14 to 20, characterized by coating sodium carboxymethyl-cellulose particles with a methacrylate alcohol solution and mixing the coated sodium carboxymethyl cellulose particles with uncoated sodium carboxymethyl cellulose particles.

22. Process according to one of the claims 14 to 20, characterized by coating sodium carboxymethyl cellulose particles with an ethyl cellulose solution and mixing the coated sodium carboxymethyl cellulose particles with uncoated sodium carboxymethyl cellulose particles.

23. Dental prosthesis adhesive according to one of claims 1 to 13, characterized in that it contains an adhesive substance selected from sodium carboxymethyl cellulose, karaya gum, guar gum, tragacanth, a polyethylene oxide polymer, a copolymer of maleic anhydride with lower alkylvinyl ether and mixed partial salts thereof, and polyacrylamide.

**Patentansprüche**

1. Haftmittel für Zahnprothesen aus Teilchen einer oder mehrerer Haftsubstanzen, dadurch gekennzeichnet, daß wenigstens ein Teil der Teilchen der Haftsubstanz(en) mit einem Überzug versehen ist, welcher sich in Speichel langsam auflöst.

2. Haftmittel für Zahnprotheses nach Anspruch 1, dadurch gekennzeichnet, daß der Überzug für die Teilchen der Haftsubstanz eine oder mehrere Substanzen, ausgewählt aus Ethylcellulose, Saccharosemonostearat, Gummi arabicum, Celluloseacetophtalat, Acrylat, Methylacrylat oder Schellack, enthält.

3. Haftmittel für Zahnprothesen nach einem der Ansprüche 1 und 2, dadurch gekennzeichnet, daß die Teilchen der Haftsubstanz wenigstens teilweise Natrium-celluloseglykolat (Carboxymethylcellulose-Natriumsalz) enthalten.

4. Haftmittel für Zahnprothesen nach einem der vorangehenden Ansprüche, dadurch gekennzeichnet, daß der Überzug wenig wasserlöslich ist.

5. Haftmittel für Zahnprothesen nach einem der Ansprüche 1—3, dadurch gekennzeichnet, daß der Überzug wasserunlöslich ist.

6. Haftmittel für Zahnprothesen nach einem der Ansprüche 1—3, dadurch gekennzeichnet, daß der Überzug bei einem pH-Wert zwischen 3,5 und 9,0 löslich ist.

7. Haftmittel für Zahnprothesen nach einem der vorangehenden Ansprüche, dadurch gekennzeichnet, daß 10—90% der verwendeten Haftsubstanz überzogen sind.

8. Haftmittel für Zahnprothesen nach Anspruch 1, dadurch gekennzeichnet, daß Natrium-celluloseglykolat überzogen mit Ethylcellulose als überzogene Haftsubstanz verwendet ist.

9. Haftmittel für Zahnprothesen nach Anspruch 8, dadurch gekennzeichnet, daß zu 50% überzogenes Natrium-celluloseglykolat als Haftsubstanz verwendet ist.

10. Haftmittel für Zahnprothesen nach einem der vorangehenden Ansprüche, dadurch gekennzeichnet, daß die wenigstens teilweise übersogenen Teilchen der Haftsubstanz(en) in eine cremige Trägersubstanz eingebettet sind.

11. Haftmittel für Zahnprothesen nach Anspruch 10, dadurch gekennzeichnet, daß die cremige Trägersubstanz Petrolatum und Flüssigpetrolatum und Paraffinöl enthält.

12. Haftmittel für Zahnprothesen nach einem der Ansprüche 1—9, dadurch gekennzeichnet, daß die wenigstens teilweise überzogenen Teilchen der Haftsubstanz(en) in eine halb-flüssige Trägersubstanz eingebettet sind.

13. Haftmittel für Zahnprothesen nach Anspruch 12, dadurch gekennzeichnet, daß die halb flüssige Trägersubstanz eine Mischung von Paraffin und Glycerin oder von Polyethylenglycol und Glycerin ist.

14. Verfahren zur Herstellung eines Haftmittels für Zahnprothesen, gekennzeichnet durch das Überziehen von Teilchen einer Haftsubstanz (von Haftsubstanzen) mit einem Überzug, welcher sich langsam in Speichel und/oder in Verdauungssekreten auflöst, und durch das Mischen der überzogenen Teilchen der Haftsubstanz(en) mit nichtüberzogenen Teilchen der Haftsubstanz(en).

15. Verfahren nach Anspruch 14, dadurch gekennzeichnet, daß die Mischung aus 90—10% der überzogenen Teilchen der Haftsubstanz und 90—10% der nicht-überzogenen Teilchen der Haftsubstanz hergestellt wird.

16. Verfahren nach Anspruch 15, dadurch gekennzeichnet, daß das Gemisch in eine Trägersubstanz eingebettet wird.

17. Verfahren nach Anspruch 16, dadurch gekennzeichnet, daß die Trägersubstanz halb-flüssig ist.

18. Verfahren nach Anspruch 17, dadurch gekennzeichnet, daß die halb-flüssige Trägersubstanz eine Mischung von Paraffin und Glycerin oder von Polyethylenglykol und Glycerin ist.

19. Verfahren nach Anspruch 17, dadurch gekennzeichnet, daß die Trägersubstanz cremig ist.

20. Verfahren nach Anspruch 19, dadurch gekennzeichnet, daß die cremige Trägersubstanz eine Mischung von Petrolatum und Flüssigpetrolatum und Paraffinöl ist.

21. Verfahren nach einem der Ansprüche 14—20, gekennzeichnet durch das Überziehen von Natrium-celluloseglykolatteilchen mit einer Methylacrylat-Alkohol-Lösung und durch das Mischen der überzogenen Natrium-celluloseglykolatteilchen mit nicht-überzogenen Natriumcelluloseglykolatteilchen.

22. Verfahren nach einem der Ansprüche 14—20, gekennzeichnet durch das Überziehen von Natrium-celluloseglykolatteilchen mit einer Ethylcelluloselösung und durch das Mischen der überzogenen Natrium-celluloseglykolatteilchen mit nicht-überzogenen Natrium-celluloseglykolatteilchen.

23. Haftmittel für Zahnprothesen nach einem der Ansprüche 1—13, dadurch gekennzeichnet, daß es eine Haftsubstanz, ausgewählt aus Natrium-celluloseglykolat, Karaya-Gummi, Guar-Mehl, Traganth, einem Polyethylenoxid-Polymer, einem Copolymer von Maleinsäureanhydrid mit einem kurzkettigen Alkyl-vinylether und gemischten, partiellen Salzen derselben, und Polyacrylamid, enthält.

**Revendications**

1. Adhésif pour prothèse dentaire formé à partir de particules d'une ou plusieurs substances adhésives, caractérisé par le fait qu'au moins une partie des particules de la substance ou des substances adhésives est munie d'un revêtement qui se dissout lentement dans la salive.

2. Adhésif pour prothèse dentaire selon la revendication 1, caractérisé par le fait que le revêtement destiné aux particules de la substance adhésive contient une ou plusieurs substances choisies entre l'éthyl-cellulose, le monostéarate de saccharose, la gomme arabique, l'acéto-phtalate, l'acrylate, le méthacrylate de cellulose ou la gomme-laque.

3. Adhésif pour prothèse dentaire selon l'une ou l'autre des revendications 1 et 2, caractérisé par le fait que les particules de la substance adhésive contiennent, au moins partiellement, de la carboxyméthyl-cellulose sodique.

4. Adhésif pour prothèse dentaire selon l'une des revendications précédentes, caractérisé par le fait que le revêtement est peu soluble dans l'eau.

5. Adhésif pour prothèse dentaire selon l'une des revendications 1 à 3, caractérisé par le fait que le revêtement est insoluble dans l'eau.

6. Adhésif pour prothèse dentaire selon l'une des revendications 1 à 3, caractérisé par le fait que le revêtement est soluble à une valeur de pH comprise entre 3,5 et 9,0.

7. Adhésif pour prothèse dentaire selon l'une des revendications précédentes, caractérisé par le fait que 10 à 90% de la substance adhésive utilisée sont revêtus.

8. Adhésif pour prothèse dentaire selon la revendication 1, caractérisé par le fait que l'on utilise, comme substance adhésive revêtue, de la carboxyméthyl-cellulose sodique revêtue d'éthylcellulose.

9. Adhésif pour prothèse dentaire selon la revendication 9, caractérisé par le fait que l'on utilise comme substance adhésive de la carboxyméthyl-cellulose sodique revêtue à 50%.

10. Adhésif pour prothèse dentaire selon l'une des revendications précédentes, caractérisé par le fait que les particules, au moins partiellement revêtues, de substance ou de substances adhésives sont noyées dans une substance support crémeuse.

11. Adhésif pour prothèse dentaire selon la revendication 10, caractérisé par le fait que la substance de support crémeuse contient du pétrolatum, de l'huile blanche et de l'huile paraffinique.

12. Adhésif pour prothèse dentaire selon l'une des revendications 1 à 9, caractérisé par le fait que les particules, au moins partiellement revêtues, de substance ou de substances adhésives sont noyées dans une substance de support semi-fluide.

13. Adhésif pour prothèse dentaire selon la revendication 12, caractérisé par le fait que la substance de support semi-fluide est un mélange de paraffine et de glycérol ou de polyéthylène-glycol et de glycérol.

14. Procédé de préparation d'un abrasif pour prothèse dentaire, caractérisé par le fait qu'il consiste à appliquer sur les particules de substance ou de substances adhésives un revêtement qui se dissout lentement dans la salive et/ou les liquides ingérés et à mélanger les particules revêtues de substance ou de substances adhésives avec des particules non revêtues de substance ou de substances adhésives.

15. Procédé selon la revendication 14, caractérisé par le fait que le mélange est préparé à partir de 90% à 10% de particules revêtues d'une substance adhésive et 90 à 10% de particules non revêtues de la substance adhésive.

16. Procédé selon la revendication 15, caractérisé par le fait que le mélange est noyé dans un support.

17. Procédé selon la revendication 16, caractérisé par le fait que le support est semi-fluide.

18. Procédé selon la revendication 17, caractérisé par le fait que le support semi-fluide est un mélange de paraffine et de glycérol ou de polyéthylène-glycol et de glycérol.

19. Procédé selon la revendication 17, caractérisé par le fait que le support est crémeux.

20. Procédé selon la revendication 19, caractérisé par le fait que le support crémeux est un mélange de pétrolatum et d'huile blanche et d'huile de paraffine.

21. Procédé selon l'une des revendications 14 à 20, caractérisé par le fait qu'il consiste à appliquer sur les particules de carboxyméthyl-cellulose sodique une solution alcoolique de méthacrylate et à mélanger les particules de carboxyméthyl-cellulose sodique revêtues avec des particules de carboxyméthyl-cellulose sodique non revêtues.

22. Procédé selon l'une des revendications 14 à 20, caractérisé par le fait qu'il consiste à appliquer aux particules de carboxyméthyl-cellulose sodique une solution d'éthyl-cellulose et à mélanger les particules de carboxyméthyl-cellulose sodique revêtues avec les particules de carboxyméthyl-cellulose sodique non revêtues.

23. Adhésif pour prothèse dentaire selon l'une des revendications 1 à 13, caractérisé par le fait qu'il contient une substance adhésive choisie entre la carboxyméthyl-cellulose sodique, la gomme de karaya, la gomme guar, la gomme adragante, un polymere du type oxyde de polyéthylène, un copolymère d'anhydride maléique et d'un éther de (alkyle inférieur) vinyle et leurs sels partiels mixtes, et un polyacrylamide.